Europäisches Patentamt

European Patent Office

Office européen des brevets

(11) Veröffentlichungsnummer: **0 518 137 A2**

# EUROPÄISCHE PATENTANMELDUNG

(21) Anmeldenummer: **92109013.0**

(22) Anmeldetag: **29.05.92**

(51) Int. Cl.5: **A61K 7/09**

(30) Priorität: **10.06.91 DE 4119044**

(43) Veröffentlichungstag der Anmeldung:
**16.12.92 Patentblatt 92/51**

(84) Benannte Vertragsstaaten:
**AT BE CH DK ES FR GB IT LI NL**

(71) Anmelder: **GOLDWELL AKTIENGESELLSCHAFT**
**Zerninstrasse 10-18**
**W-6100 Darmstadt 13(DE)**

(72) Erfinder: **Burkhard, Rose**
**Katharinenstrasse 14**
**W-6100 Darmstadt 13(DE)**
Erfinder: **Tennigkeit, Jürgen, Dr.**
**Felsbergstrasse 51**
**W-6104 Seeheim 2(DE)**

(54) Verfahren zur dauerhaften Verformung von menschlichen Haaren.

(57) Eine schonende Verformung menschlichen Haares wird durch die Einhaltung folgender Verfahrensschritte erzielt:

Einwirkung eines Reduktionsmittels üblicher Art auf Basis mindestens einer Thioverbindung; ohne vorheriges Ausspülen "Vorfixierung" mit einer mindestens eine Dithioverbindung enthaltenden Zusammensetzung; Endfixierung mit einer ein Oxidationsmittel, insbesondere Wasserstoffperoxid, in niedriger Konzentration enthaltenden Zusammensetzung.

EP 0 518 137 A2

Die vorliegende Erfindung betrifft ein verfahren zur dauerhaften verformung von menschlichen Haaren, das die Eigenschaften des dauergewellten Haares verbessert.

Bekanntlich erfordert die Dauerwellung im Prinzip zwei Behandlungsschritte:

Die reduktive Spaltung der Cystin-Disulfidbrücken des Haares durch Einwirkung eines Reduktionsmittels und die anschließende Fixierung durch Aufbringung eines Oxidationsmittels, wodurch die Cystin-Disulfidbrücken wieder hergestellt werden.

Das klassische Reduktionsmittel ist, wie bereits aus den Pionierpatenten DE-PS 948 186 und 972 424 hervorgeht, dabei die Thioglykolsäure, z. B. als Ammonium- oder Monoethanolaminsalz, die in den letzten Jahren durch Glycerinmonothioglykolat teilweise ersetzt wurde; jedoch werden auch Thiomilchsäure und deren Ester sowie auch anorganische Sulfite eingesetzt.

Die Thioglykolsäure enthaltenden Zusammensetzungen weisen dabei einen pH-Wert im Bereich zwischen etwa 7,5 und etwa 9,0, insbesondere 8,5 bis 9,0 auf, wobei die Alkalisierung heute in der Regel, neben Ammoniak, durch Zusatz von Ammonium-(bi)-carbonat erzielt wird.

Die wiederholte Einwirkung stark alkalischer Reduktionsmittel und darauffolgende Fixierung mit Oxidationsmitteln kann insbesondere auch bei nicht ganz intaktem, d. h. durch Bleichung oder alkalische Oxidationsfärbung vorgeschädigtem Haar, eine weitere Haarschädigung bewirken.

Gleiches gilt für die Fixierung, bei der in der Regel Oxidationsmittel-Konzentrationen von etwa 2 bis 4 Gew.-%, berechnet auf Wasserstoffperoxid, zum Einsatz gelangen.

Die vorliegende Erfindung geht daher von der Aufgabenstellung aus, ein Verfahren zur dauerhaften Verformung von menschlichen Haaren zu schaffen, das zu einer schonenden aber wirksamen Verformung des Haares führt, wobei Spannkraft und Elastizität des dauergewellten Haares erhalten bleiben.

Diese Aufgabe wird dadurch gelöst, daß auf das Haar eine mindestens eine Thioverbindung enthaltende Reduktionsmittel-Zusammensetzung in an sich bekannter Weise zur Einwirkung gebracht und anschließend, ohne vorheriges Ausspülen, eine Vorfixierung durch Behandlung mit einer mindestens eine Dithioverbindung enthaltenden Zusammensetzung durchgeführt, das so behandelte Haar gespült und anschließend in an sich bekannter weise mit einer Oxidationsmittel-Zusammensetzungendgültig fixiert wird, wobei die Konzentration des Oxidationsmittels, berechnet auf Wasserstoffperoxid, bei 0,5 bis 1,5 Gew.-% der Gesamtzusammensetzung liege.

Auf diese Weise wird ein wesentlich elastischeres Haar erhalten als es bei der Anwendung konventioneller Verfahren der Fall ist. Mutmaßlich wird durch die Vorfixierung mit Dithioverbindungen bereits ein wesentlicher Teil der gespaltenen Disulfidbrücken im Haar wieder hergestellt.

Aus der DE-A 37 07 415 ist ein Verfahren zur dauerhaften Haarverformung bekannt, wo unmittelbar vor der Anwendung ein Gemisch aus einem zuvor voneinander getrennt gehaltenen Zweikomponentenpräparat hergestellt wird, von dem die eine Komponente einen Thioglykolsäureester und die andere eine wäßrige Lösung von Dithiodiglykolsäure, Dithiodimilchsäure bzw. deren Salzen in wäßriger Lösung zusammen mit Alkali- oder Ammoniumbicarbonat enthält. Das mehrstufige Verfahren nach der Erfindung wird dadurch nicht berührt.

In der DE-A 22 63 203 ist ein Verfahren zur Dauerwellung menschlicher Haare beschrieben, wobei das Haar in erster Stufe mit einem Verformungsmittel auf Basis von Thioglykolsäure- oder Thiomilchsäuresalzen behandelt und nach einer gewissen Einwirkungszeit in einer zweiten Stufe mit einem weiteren Anteil des Verformungsmittels behandelt wird, dem ein hydrophiler Thioglykolsäure- bzw. Thiomilchsäureester zugesetzt wurde. Die mit dem erfindungsgemäßen Verfahren erzielbaren Effekte werden mit diesem bekannten Verfahren nicht erreicht.

Die Reduktionsmittel werden in Form von wässrigen Lösungen, Gelen, Emulsionen (Cremes) oder auch als Aerosolschäume eingesetzt und enthalten, neben dem Reduktions- und Alkalisierungmittel, haarkonditionierende Substanzen, beispielsweise kationische Polymere, gegebenenfalls Verdickungsmittel, sogenannte Carrier, die insbesondere auch die Penetration des Produktes erhöhen, Komplexbildner, Trübungsmittel, Duftstoffe und, zur Verbesserung des Netz- und Penetrationsvermögens, auch oberflächenaktive Substanzen.

Wesentlicher Bestandteil der Fixier- bzw. Neutralisationmittel sind Oxidationsmittel. Das am häufigsten benutzte Oxidationmittel ist Wasserstoffperoxid, dessen Konzentration im erfindungsgemäß eingesetzten Fixierungsmittel zwischen etwa 0,5 und etwa 1,5 Gew.-% und damit weit unterhalb der bisher üblichen Einsatzkonzentration liegt.

Weitere zum Einsatz gelangende Oxidationsmittel sind Alkalibromate, Harnstoffperoxid und Natriumperborat, die beiden letzgenannten selbstverständlich in wasserfreien Produkten.

Diese Zusammensetzungen werden vorzugsweise in Form wässriger Lösungen oder als Aerosolschäume, seltener als Gele, konfektioniert.

Sie können verschiedene weitere Bestandteile wie Stabilisatoren, Pflanzenextrakte, haarkonditionierende

2

Wirkstoffe und Tenside enthalten.

Als reduzierende Wirkstoffe in den erfindungsgemäß zum Einsatz gelangenden Dauerwellmitteln werden, wie bereits erwähnt, neben anorganischen Sulfiten wie Natriumbisulfit, insbesondere Thioglykolsäure und Ammoniumthioglykolat, Thiomilchsäure, deren Salze und Ester, 2-Mercaptoethylamin, Cystein und dessen Hydrochlorid, Cysteamin, N-Acetylcystein, Thioessigsäure, deren Salze und Ester sowie insbesondere Thioglykolsäuremonoglycerinester eingesetzt. Im Falle von dessen Verwendung oder der Verwendung ähnlicher Thioester erfolgt die Vermischung mit der restlichen Reduktionsmittel-Zusammensetzung unmittelbar vor der Applikation.

Die Anwendungskonzentration des reduzierenden Wirkstoffs liegt in Abhängigkeit von der Struktur desselben im allgemeinen zwischen etwa 2,5 und etwa 15 Gew.-% der Reduktionsmittel-Zusammensetzung, vorzugsweise zwischen etwa 3 und etwa 10 Gew.-%.

Die Menge an Alkalisierungsmittel ist abhängig vom reduzierenden Wirkstoff. Vorzugsweise enthält die Reduktionsmittel-Zusammensetzung etwa 1 bis etwa 10, insbesondere etwa 2 bis etwa 8 Gew.-% desselben.

Bevorzugtes Alkalisierungsmittel im Rahmen der Erfindung ist Ammoniumcarbamat, das alleine oder in Kombination mit weiteren Alkalisierungsmitteln wie Ammoniak und/oder Ammonium(bi)carbonat eingesetzt wird. Es wird die Einstellung eines pH-Wertes im Bereich zwischen etwa 7 und etwa 8 angestrebt.

Die erfindungsgemäß zum Einsatz kommenden Dauerwellmittel enthalten vorzugsweise auch Tenside. Deren Anteil liegt bei 0,1 bis etwa 10, insbesondere etwa 1 bis etwa 5 Gew.-%.

Sowohl bei den in den Reduktionsmittel-Zusammensetzungen als auch bei den in den Fixiermitteln eingesetzten Tensiden handelt es sich vorzugsweise um die bekannten anionaktiven Produkte, die gegebenenfalls auch in Kombination mit nichtionischen Tensiden zum Einsatz gelangen.

Geeignete anionische Tenside sind insbesondere die bekannten Alkylethersulfate und -carbonsäuren, insbesondere in Form ihrer Alkalisalze, sowie Eiweiß-Fettsäure-Kondensate.

Geeignete nichtionische Tenside sind insbesondere C8-C18-Fettalkoholpolyglykolether, Fettsäurepolyglykolester, Fettsäurealkanolamide, Aminoxide und vor allem C8-C18-Alkylpolyglykoside.

Es können auch amphotere Tenside wie die bekannten Betaine und Amidobetaine sowie, insbesondere in kationischen Fixierungen, kationaktive Tenside wie quaternäre Ammoniumverbindungen eingesetzt werden.

Ein weiterer wünschenswerter Bestandteil der erfindungsgemäß verwendeten Reduktionsmittel-Zusammensetzungen ist ein $C_3$-$C_6$-Alkandiol bzw. dessen Ether, insbesondere Mono-$C_1$-$C_3$-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen etwa 1 und etwa 30, vorzugsweise etwa 2,5 bis etwa 15, insbesondere etwa 5 bis etwa 10 Gew.-% der Reduktionsmittel-Zusammensetzung.

Neben den $C_3$-$C_6$-Alkandiolen bzw. deren Ethern können zusätzlich auch Propylencarbonat (4-Methyl-1,3-dioxolan-2-on), N-Alkylpyrrolidone, Glycerin und Harnstoff Verwendung finden.

Die erfindungsgemäß eingesetzten Mittel können selbstverständlich alle in Dauerwellmitteln üblichen Stoffe enthalten, auf deren detaillierte Aufzählung hier verzichtet wird.

Zur Vermeidung von Wiederholungen wird vielmehr auf den Stand der Technik verwiesen, wie er beispielsweise in "Ullmann's Encyclopedia of Industrial Chemistry", Vol. A12 (1986), S.588 bis 591, sowie insbesondere der Monographie von K. Schrader, "Grundlagen und Rezepturen der Kosmetika", 2. Aufl. (1989, Hüthig-Verlag), S.823 bis 840, sowie in dem Übersichtsartikel von D. Hollenberg et al in "Seifen-Öle-Fette-Wachse" 117 (1981), S.81-87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Bei den in den erfindunggemäß zur Herstellung der Vorfixierung verwendeten Zusammensetzungen kommen als Dithioverbindungen vorzugsweise Dithiodiglykolsäure, Diothiodimilchsäure sowie deren Ester und Alkali- bzw. Ammoniumsalze in Betracht, vorzugsweise in einer Menge zwischen etwa 2,5 und etwa 10, vorzugsweise etwa 4 bis 7 Gew.-% der Zusammensetzung.

Als weitere Dithioverbindung kann gegebenenfalls auch Cystamin zum Einsatz gelangen.

Falls erwünscht, kann vor dem Auftrag des Reduktionsmittels noch ein Vorbehandlungsmittel appliziert werden, wie es beispielweise in dem DE-A 37 40 926 beschrieben ist. Nach dem Aufbringen dieses Vorbehandlungsmittels wird das Haar aufgewickelt und die Reduktionsmittelzusammensetzung aufgetragen. Nach etwa 15- bis 20-minütiger Einwirkung wird, ohne Spülung, das mindestens eine Dithioverbindung enthaltende Vorfixiermittel aufgetragen, dessen durchschnittliche Einwirkzeit etwa 3 bis 8, vorzugsweise etwa 5 Minuten beträgt.

Dieses Verfahren bietet auch die Möglichkeit, das Zwischen- bzw. Vorfixiermittel je nach Notwendigkeit unterschiedlich für die einzelnen Haarsträhnen zu dosieren. Anschließend wird gespült und auf übliche Weise mit einem Fixiermittel auf Basis eines Oxidationsmittels wie Wasserstoffperoxid in niedriger Konzentration fixiert.

Die folgenden Ausführungsbeispiele dienen der weiteren Illustration der Erfindung.

Beispiel 1

Auf mit einem konventionellen, mild alkalischen handelsüblichen Dauerwellmittel behandeltes Haar wird eine Lösung der folgenden Zusammensetzung aufgebracht:

| | |
|---|---|
| Dithiodiglykolsäure, Diammoniumsalz (40%-ig) | 12,50 (Gew.-%) |
| Glycerin | 5,00 |
| Ammoniumbicarbonat | 0,50 |
| Laurylpolyglykolether | 0,50 |
| Parfum | 0,20 |
| Demineralisiertes Wasser | ad 100,00 |

Nach fünfminütiger Einwirkungsdauer wird sorgfältig gespült und anschließend mit der folgenden Oxidationsmittel-Zusammensetzung "endfixiert":

| | |
|---|---|
| Wasserstoffperoxid | 1,00 (Gew.-%) |
| Natriumlauryldiglykolethersulfat | 5,00 |
| Phenacetin | 0,02 |
| Phosphorsäure zur Einstellung auf pH 3,5 | q.s. |
| Demneralisiertes Wasser | ad 100,00 |

Es wird ein glänzendes, gut dauergewelltes Haar mit gegenüber konventionell dauergewelltem Haar meßbar verbesserter Elastizität und Spannkraft erhalten.

Beispiel 2

Auf mit einem konventionellen, schwach sauren Dauerwellmittel auf Basis Glycerinmonothioglykolsäureester dauergewelltes Haar wird die folgende Zusammensetzung aufgebracht:

| | |
|---|---|
| Diammoniumdithiodiglykolat(40%-ig) | 8,75 (Gew.-%) |
| 1,2-Propandiol | 5,00 |
| Dinatriumhydrogenphoshat | 0,50 |
| PEG-40-hydriertes Ricinusöl | 0,50 |
| Eiweißhydrolysat | 1,00 |
| Parfum | 0,20 |
| Wasser | ad 100,00 |

Nach etwa vierminütiger Einwirkungszeit wird gut mit Wasser gespült und anschließend mit folgender Zusammensetzung in üblicher Weise "endfixiert":

| Wasserstoffperoxid | 1,20 (Gew.-%) |
|---|---|
| Kationisches Polymer (Polyquaternium[R]-10) | 0,25 |
| Cocosaminoxid | 1,00 |
| Phenacetin | 0,02 |
| Nonylphenolpolyglykolether | 0,50 |
| Parfum | 0,10 |
| Phosphorsäure zur pH-Einstellung auf 2,8 | q.s. |
| Demineralisiertes Wasser | ad 100,00 |

Es wird ein gut gewelltes Haar erhalten, dessen Elastizität und Spannkraft weitgehend erhalten geblieben sind.

Beispiel 3

Die folgende Zusammensetzung ist als "Vorfixierung" im Sinne der Erfindung in Kombination mit üblichen Dauerwellmitteln geeignet:

| Diammoniumdithiodiglykolat(40%-ig) | 4,50 (Gew.-%) |
|---|---|
| Ethylcarbitol | 7,50 |
| Ammoniumbicarbonat | 1,00 |
| Nonylphenolpolyglykolether | 1,00 |
| Kationisches Polymer (Polyquaternium[R]-10) | 0,10 |
| Parfum | 0,30 |
| Demineralisiertes Wasser | ad 100,00 |

Die "Endfixierung" kann mit folgender Zusammensetzung erfolgen:

| Wasserstoffperoxid | 0,90 (Gew.-%) |
|---|---|
| Phenacetin | 0,02 |
| Parfum | 0,10 |
| Natriumlaurylsulfat | 2,50 |
| Phosphorsäure zur pH-Einstellung auf 3,5 | q.s. |
| Demineralisiertes Wasser | ad 100,00 |

Nachfolgend wird noch eine charakteristische Rezeptur für eine im Rahmen der Erfindung besonders geeignete Reduktionsmittel-Zusammensetzung gegeben:

| Thioglykolsäure (80%-ig) | 10,00 (Gew.-%) |
|---|---|
| Ammoniumcarbamat | 6,00 |
| Ammoniak | 1,10 |
| Cocosamidopropylbetain | 2,00 |
| 1,2-Propandiol | 5,00 |
| Parfum | q.s. |
| Wasser | ad 100,00 |

**Patentansprüche**

1. Verfahren zur dauerhaften Verformung von menschlichen Haaren, gekennzeichnet durch eine Kombination folgender Schritte:

    a) Aufbringen und Einwirkung einer mindestens eine Thioverbindung und ein Alkalisierungsmittel enthaltenden Reduktionsmittel-Zusammensetzung;

    b) Aufbringen einer mindestens eine Dithioverbindung enthaltenden Zusammensetzung ("Vorfixierung");

c) endgültige Fixierung durch Behandlung mit einer Oxidationsmittel-Zusammensetzung, wobei die Konzentration des Oxidationsmittels, berechnet auf Wasserstoffperoxid, etwa 0,5 bis 1,5 Gew.-% der Zusammensetzung beträgt.

2. Verfahren nach Anspruch 1, dadurch gekennzeichnet, daß die Reduktionsmittelusammensetzung 2,5 bis 15 Gew.-%, bezogen auf die zur Anwendung kommende Gesamtzusammensetzung, Thioglykolsäure, Thioessigsäure, Thiomilchsäure und/oder deren Ammoniumsalze, Thioglykolsäureglycerinester, Thiomilchsäureglycerinester, Cystein, N-Acetylcystein, Cysteamin, 2-Mercaptoethylamin und/oder anorganische Sulfite, insbesondere Natrium- oder Ammoniumbisulfit und Gemische aus zwei oder mehreren dieser Stoffe als Thioverbindungen enthält.

3. Verfahren nach Anspruch 1 oder 2, dadurch gekennzeichnet, daß das Alkalisierungmittel zumindest teilweise aus Ammoniumcarbamat besteht.

4. Verfahren nach einem der vorangehenden Ansprüche, dadurch gekennzeichnet, daß die die Vorfixierung bewirkende Zusammensetzung etwa 2,5 bis 10 Gew.-%, bezogen auf die Zusammensetzung, Dithiodiglykolsäure, Dithiodimilchsäure, deren Ester und/oder deren Alkali- oder Ammoniumsalze als Dithioverbindung enthält.

5. Verfahren nach Anspruch 4, dadurch gekennzeichnet, daß die die Vorfixierung bewirkende Zusammensetzung etwa 4 bis etwa 7 Gew.-%, bezogen auf die Zusammensetzung, der Dithioverbindung enthält.